# EUROPEAN PATENT APPLICATION

(11) **EP 2 422 629 A1**
(43) Date of publication of application: **29.02.2012**
(21) Application number: 10380107.2
(22) Date of filing: 23.08.2010
(51) Int. Cl.: A23L 1/09, A23L 1/29, A61K 31/715, A61K 31/718

(54) **Methods for enhancing cognition and/or memory using maltodextrins**

(71) Applicant: Abbott Laboratories, Columbus OH 43219 (US)
(72) Inventor: BARRANCO PEREZ, Alejandro, 18195 Cullar-Vega (Granada) (ES); RAMIREZ GONZALEZ, Maria, 18003 Granada (ES); RUEDA CABRERA, Ricardo, 18008 Granada (ES)
(74) Representative: Ungria Lopez, Javier

(57) **Abstract**

Disclosed are nutritional compositions and methods for enhancing the cognition and/or improving the memory of an infant or child. The methods include administering to an infant or child a nutritional composition comprising maltodextrin such that a single dose of maltodextrin is sufficient to enhance cognition and/or improve memory in the infant or child after the administration. The nutritional composition may include liquid nutritional compositions or solid nutritional products and may provide the sole source of nutrition or may be administered as a supplemental nutritional source.

## Description

### TECHNICAL FIELD

The present disclosure relates to methods for enhancing cognition and/or improving the memory of a subject, and in particular, an infant or child, that include administering a nutritional composition including maltodextrin as a carbohydrate source. The nutritional composition is administered such that a single dose of maltodextrin is sufficient for enhancing cognitive and memory performance in the infant or child after administration.

### BACKGROUND OF THE DISCLOSURE

Infant formulas are well known and readily available as a sole or supplemental feeding source for infants. A number of cow or soy-protein based formulations are available and also generally contain at least a fat source, a carbohydrate source, vitamins and minerals. The carbohydrate source has generally been lactose or sucrose-based, although other carbohydrates have been utilized.

Maltodextrins have also been conventionally utilized in nutritional products as a carbohydrate and source of energy. Specifically, maltodextrins are known to be hydrolyzed in the intestinal lumen to glucose that is readily absorbed, thereby contributing to the rise in blood glucose levels. In fact, maltodextrins have been shown to increase blood glucose to higher levels than sucrose. This is believed to be due in part as the fructose portion of sucrose does not contribute to the glycemic index.

It has now unexpectedly been discovered that an enhancement in cognition and/or memory in an infant or child can be achieved by specifically administering a nutritional composition including maltodextrin as the carbohydrate source. While previous studies have shown improved memory of a subject after administration of glucose, the results of the methods used herein are surprisingly different. Specifically, while memory improvement is typically seen a half hour to an hour after administration of glucose, memory improvement after administration of the nutritional compositions including maltodextrin as described herein are seen after three hours, when the blood glucose peak has already gone.

Additionally, it has been found that the nutritional compositions including maltodextrin may be administered prior to a cognitive task and improved cognitive results are still shown in the infant or child. Previous glucose studies show improvement only when the glucose is administered immediately after the cognitive task. Additionally, the memory improving effects of glucose have only been shown when glucose is administered intraperitoneally. By orally administering the nutritional composition including maltodextrin, however, the cognition and/or memory of the infant or child is improved.

### SUMMARY OF THE DISCLOSURE

One embodiment is directed to a method of enhancing cognition in an infant. The method comprises administering to the infant a nutritional composition comprising maltodextrin. The nutritional composition is administered such that the intake of maltodextrin is from about 0.5 g/kg body weight/dose to about 2.5 g/kg body weight/dose.

Another embodiment is directed to a method of improving memory in an infant. The method comprises administering to the infant a nutritional composition comprising maltodextrin. The nutritional composition is administered such that the intake of maltodextrin is from about 0.5 g/kg body weight/dose to about 2.5 g/kg body weight/dose.

Another embodiment is directed to a method of enhancing cognition in a child. The method comprises administering to the child a nutritional composition comprising maltodextrin. The nutritional composition is administered such that the intake of maltodextrin is from about 0.5 g/kg body weight/dose to about 2.5 g/kg body weight/dose.

Yet another embodiment is directed to a method of improving memory in a child. The method comprises administering to the child a nutritional composition comprising maltodextrin. The nutritional composition is administered such that the intake of maltodextrin is from about 0.5 g/kg body weight/dose to about 2.5 g/kg body weight/dose.

Another embodiment is directed to a method of enhancing cognition and/or improving memory in an infant or child comprising administering to the infant or child a nutritional composition comprising maltodextrin, wherein maltodextrin is the sole source of carbohydrate in the nutritional composition. The nutritional composition is administered such that the intake of maltodextrin is from about 0.5 g/kg body weight/dose to about 2.5 g/kg body weight/dose.

Still another embodiment is directed to a method of enhancing cognition and/or improving memory in an infant or child, the method comprising administering to the infant or child once per day a nutritional composition comprising maltodextrin. The nutritional composition is administered such that the intake of maltodextrin is from about 0.5 g/kg body weight/dose to about 2.5 g/kg body weight/dose.

Another embodiment is directed to a method of enhancing cognition and/or improving memory in an infant or child receiving multiple feedings per day. The method comprises administering to the infant or child during a first feeding a nutritional composition comprising maltodextrin. The nutritional composition is administered such that the intake of maltodextrin is from about 0.5 g/kg body weight/dose to about 2.5 g/kg body weight/dose. Administration of the nutritional composition during the first feeding produces an effect in the infant or child more than one hour after administration of the nutritional composition, the effect being selected from the group consisting of enhanced cognition, improved memory, and combinations thereof. The method further comprises administering to the infant or child the nutritional composition during a second feeding such that the intake of maltodextrin is from about 0.5 g/kg body weight/dose to about 2.5 g/kg body weight/dose. The second feeding is administered after the effect produced by the first feeding has diminished.

It has now been discovered that the administration of maltodextrin to an infant or child may enhance the cognition and/or memory of the infant or child. Specifically, a nutritional composition including maltodextrin is administered orally prior to a cognitive task. Following administration, the infant or child shows improved cognition and/or memory, even once blood glucose levels have returned to fasting levels.

### BRIEF DESCRIPTION OF THE DRAWINGS

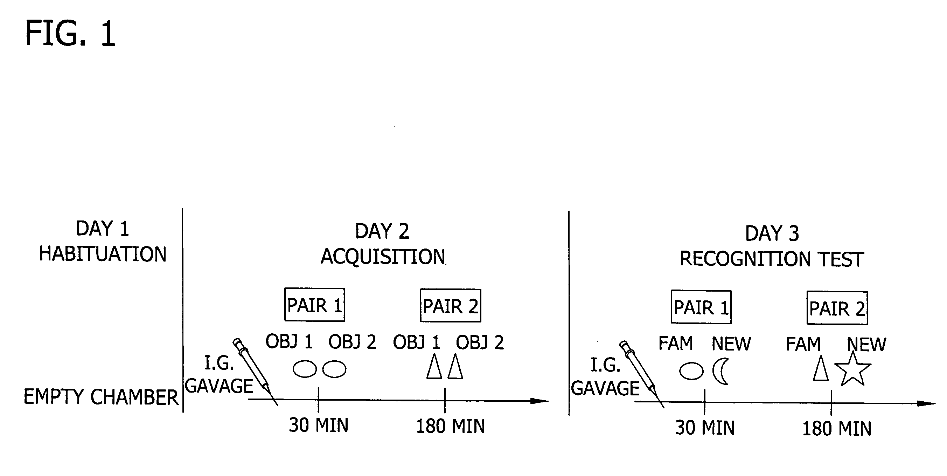

**Figure 1** depicts the object recognition memory behavioral procedure used in Examples 1, 2 and 3.

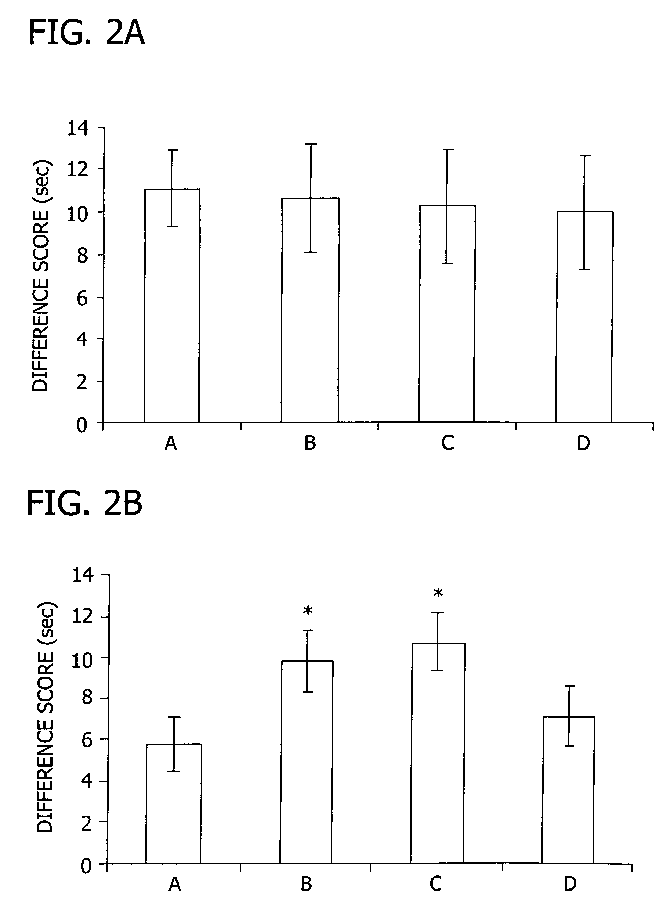

**Figure 2** depicts the mean (+SEM) difference score of the time (sec) spent exploring the novel object and the time spent exploring the familiar object 30 minutes (Figure 2A) and 180 minutes (Figure 2B) after the administration of the test solution in the testing session as in Example 1. Group A: Sucrose; Group B: Maltodextrin DE 19-24; Group C: Maltodextrin DE 8-10; and Group D: Water. *: significantly different at p<0.05 from group A.

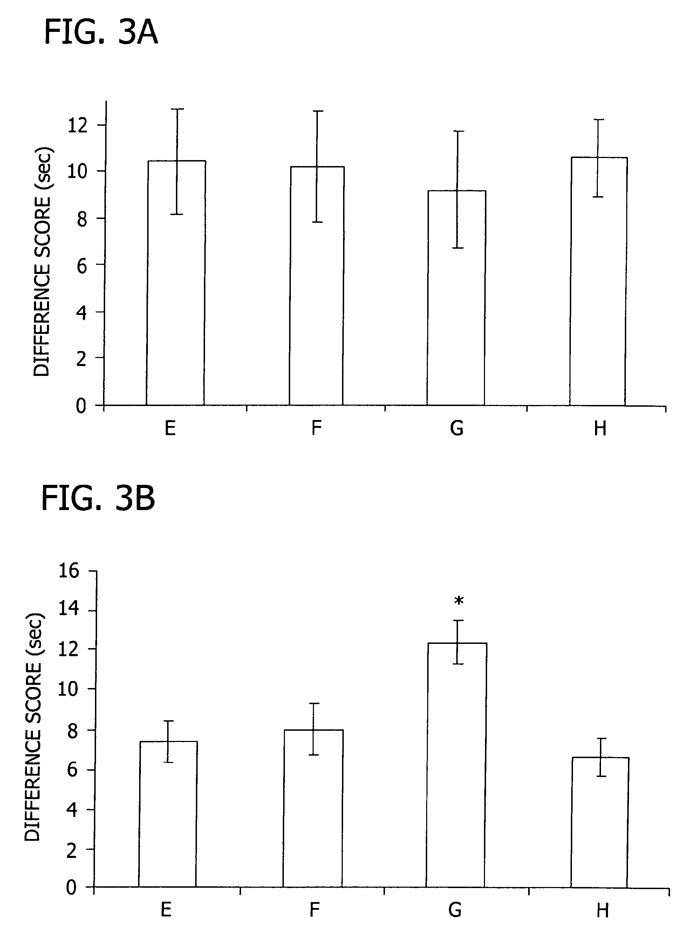

**Figure 3** depicts the mean (+SEM) difference score of the time (sec) spent exploring the novel object and the time spent exploring the familiar object 30 minutes (Figure 3A) and 180 minutes (Figure 3B) after the administration of the test solution in the testing session as in Example 2. Group E: γ-Cyclodextrin; Group F: Sucromalt; Group G: Maltodextrin DE 8-10; and Group H: Sucrose. *: significantly different at p<0.05 from all the groups.

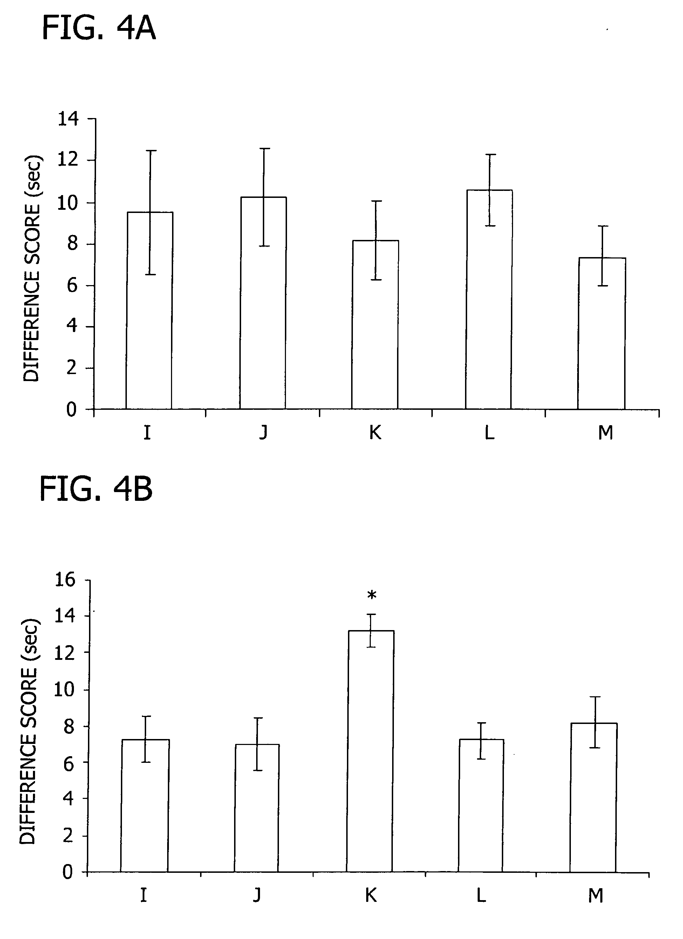

**Figure 4** depicts the mean (+SEM) difference score of the time (sec) spent exploring the novel object and the time spent exploring the familiar object 30 minutes (Figure 4A) and 180 minutes (Figure 4B) after the administration of the test solution in the testing session as in Example 3. Group I: Water; Group J: Glucose; Group K: Maltodextrin DE 8-10 before acquisition and testing sessions; and Group L: Maltodextrin DE 8-10 before acquisition session; Group M: Maltodextrin DE 8-10 before testing session. *: significantly different at p<0.05 from all the groups.

### DETAILED DESCRIPTION OF THE DISCLOSURE

The methods disclosed herein are directed to methods utilizing nutritional compositions comprising maltodextrin for enhancing cognition and/or memory in an infant or child. The essential or optional elements or features of the various embodiments are described in detail hereinafter.

The term "nutritional composition" means the referenced material comprises at least one of fat, protein, and carbohydrate and is suitable for oral administration to a human. The nutritional composition may further comprise vitamins, minerals and other ingredients and represent a sole, primary, or supplemental source of nutrition.

The term "infant" as used herein, unless otherwise specified, refers to children not more than about one year of age, and includes infants from 0 to about 12 months of age, low birth weight infants at less than 2,500 grams at birth, and preterm infants born at less than about 37 weeks gestational age, typically from about 26 weeks to about 34 weeks gestational age.

The term "child" or "children" as used herein refers to children not more than 12 years of age, and includes children from about 12 months to about 12 years of age. The term "adult" as used herein refers to adults and children about 12 years and older.

The term "infant composition" or "infant formula" as used herein, unless otherwise specified, refers to a nutritional composition designed for infants that contains sufficient nutrients such as proteins, carbohydrates, lipids, vitamins, and minerals to potentially serve as a supplemental, primary, or sole source of nutrition.

The term "ready-to-feed" as used herein, unless otherwise specified, refers to nutritional compositions in liquid form suitable for administration, including reconstituted powders, diluted concentrates, and manufactured liquids.

All percentages, parts and ratios as used herein are by weight of the total composition, unless otherwise specified. All such weights as they pertain to listed ingredients are based on the active level and, therefore, do not include solvents or by-products that may be included in commercially available materials, unless otherwise specified.

All numerical ranges as used herein, whether or not expressly preceded by the term "about", are intended and understood to be preceded by that term, unless otherwise specified.

The compositions and methods herein may also be free of any optional or other ingredient or feature described herein provided that the remaining composition still contains the requisite ingredients or features as described herein. In this context, the term "free" means the selected composition or method contains or is directed to less than a functional amount of the ingredient or feature, typically less than 0.1 % by weight, and also including zero percent by weight, of such ingredient or feature.

Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 2 to 8, from 3 to 7, from 5 to 6, from 1 to 9, from 3.6 to 4.6, from 3.5 to 9.9, and so forth.

Any reference to singular characteristics or limitations of the present disclosure shall include the corresponding plural characteristic or limitation, and vice versa, unless otherwise specified or clearly implied to the contrary by the context in which the reference is made.

Any combination of method or process steps as used herein may be performed in any order, unless otherwise specifically or clearly implied to the contrary by the context in which the referenced combination is made.

The nutritional compositions and methods may comprise, consist of, or consist essentially of the elements and features of the disclosure described herein, as well as any additional or optional ingredients, components, or features described herein or otherwise useful in a nutritional application.

### Product Form

The nutritional compositions utilized in the methods of the present disclosure may be formulated in any known or otherwise suitable product form for oral or parenteral administration. Oral product forms are preferred and include any solid, liquid, or powder formulation suitable for use herein, provided that such a formulation allows for safe and effective oral delivery of the essential and other selected ingredients from the selected product form.

Non-limiting examples of solid nutritional product forms suitable for use herein include powdered infant formulas, powdered toddler formulas, powdered human milk fortifiers, snack and meal replacement products, including those formulated as bars, sticks, cookies or breads or cakes or other baked goods, frozen liquids, candy, breakfast cereals, powders or granulated solids or other particulates, snack chips or bites, frozen or retorted entrees and so forth. Generally preferred solid nutritional products for use with the methods of the present disclosure include powdered infant formulas and powdered toddler formulas.

Non-limiting examples of liquid product forms suitable for use herein include liquid infant formulas (including both ready-to-feed formulations, concentrated formulations, and reconstituted powders), liquid toddler formulas (including both ready-to-feed formulations, concentrated formulations, and reconstituted powders), liquid human milk fortifiers (including both ready-to-feed and concentrated formulations), snack and meal replacement products, hot or cold beverages, carbonated or non carbonated beverages, juices or other acidified beverages, milk or soy-based beverages, shakes, coffees, teas, enteral feeding compositions, and so forth. These liquid compositions are most typically formulated as suspensions or emulsions, but can also be formulated in any other suitable form such as solutions, liquid gels, and so forth.

Other non-limiting examples of suitable oral product forms include semi-solid or semiliquid compositions (e.g., puddings, gels), as well as more conventional product forms such as capsules, tablets, caplets, pills, and so forth.

The nutritional compositions may be formulated to include solely maltodextrin or a source thereof as described herein, or alternately, may be combined with any number of optional ingredients to form a number of different product forms. The nutritional compositions of the present disclosure are preferably formulated as a nutritional composition, which may include a liquid or powdered nutritional or infant formula, a liquid or powdered human milk fortifier, or solid matrix nutritionals, among others.

The nutritional compositions of the present disclosure may have any caloric density suitable for the targeted or intended patient population, i.e., infants or children, or provide such a density upon reconstitution of a powder embodiment or upon dilution of a liquid concentrate embodiment. For example, most common caloric densities for the infant formula embodiments of the present disclosure are generally at least about 19 kcal/fl oz (660 kcal/liter), more typically from about 20 kcal/f1 oz (675-680 kcal/liter) to about 25 kcal/f1 oz (820 kcal/liter), even more typically from about 20 kcal/fl oz (675-680 kcal/liter) to about 24 kcal/f1 oz (800-810 kcal/liter). Generally, the 22-24 kcal/f1 oz formulas are more commonly used in preterm or low birth weight infants, and the 20-21 kcal/fl oz (675-680 to 700 kcal/liter) formulas are more often used in term infants. Children and adult nutritional formulas may have any caloric density suitable for the targeted or intended population.

For product forms such as lozenges, tablets (e.g. chewable, coated, etc.) pastes, or gels, the maltodextrin may be formulated at concentrations most typically ranging from about 30% to about 85%, including from about 40% to about 70%, including from about 45% to about 65%, and also including about 50%, by weight of the product form, all in combination with excipients or other ingredients such as acidulants, flavors, and colors.

### Maltodextrin

The nutritional compositions and methods of the present disclosure utilize maltodextrin as a carbohydrate source. In one embodiment of the present disclosure an amount of maltodextrin is included in a nutritional composition so as to enhance cognition. In another embodiment, maltodextrin is included in a nutritional composition to improve memory.

Suitable maltodextrins for use in the nutritional compositions and methods of the present disclosure include both powdered and liquid forms of maltodextrin. Specific examples of suitable maltodextrins include DE 19-24 RMS 55832 and DE 10 (8-10) RMS1136, both commercially available from Cerestar Ibérica (Spain). In some embodiments, maltodextrin is the sole source of carbohydrate in the nutritional composition. Alternately, other carbohydrates in addition to maltodextrin may also be included in the nutritional composition.

Typically, the amount of maltodextrin included in the nutritional compositions of the present disclosure is at least about 30% (by weight nutritional composition). In some embodiments the amount of maltodextrin is from about 30% (by weight nutritional composition) to about 85% (by weight nutritional composition), including from about 40% (by weight nutritional composition) to about 70% (by weight nutritional composition), including from about 45% (by weight nutritional composition) to about 65% (by weight nutritional composition), and also including about 50% (by weight nutritional composition).

Typically, the maltodextrin is included in the nutritional compositions as described herein in sufficient amounts as to provide the user with from about 0.5 g/kg body weight/dose to about 2.5 g/kg body weight/dose maltodextrin, or from about 0.6 g/kg body weight/dose to about 2.0 g/kg body weight/dose maltodextrin, or from about 0.75 g/kg body weight/dose to about 1.75 g/kg body weight/dose maltodextrin, or from about 0.9 g/kg body weight/dose to about 1.25 g/kg body weight/dose maltodextrin, or about 1 g/kg body weight/dose maltodextrin. As used herein, the term "dose" refers to the amount of nutritional composition consumed by an individual at each feeding. It will be recognized by one skilled in the art based on the disclosure herein that the exact amount of maltodextrin required by a specific individual subject to receive the benefits described herein will depend upon a number of factors including, for example, the specific physical condition and age of the individual.

### Macronutrients

The compositions of the present disclosure may further comprise one or more macronutrients including a fat source and a protein source, all in addition to the maltodextrin as defined herein.

The optional macronutrients in combination with the other essential or added ingredients may provide up to about 1000 kcal of energy per serving or dose, including from about 25 kcal to about 900 kcal, also including from about 75 kcal to about 700 kcal, also including from about 100 kcal to about 500 kcal, also including from about 150 kcal to about 400 kcal, and also including from about 200 kcal to about 300 kcal, per serving or dose, preferably as a single, undivided serving or dose.

Many different sources and types of proteins and lipids are known and can be used in the various products described herein, provided that the selected nutrients are safe and effective for oral administration and are compatible with the essential and other added ingredients.

Proteins suitable for use in the compositions of the present disclosure, in addition to the maltodextrin component as described herein, include hydrolyzed, partially hydrolyzed or non-hydrolyzed proteins or protein sources, and can be derived from any known or otherwise suitable source such as milk (e.g., casein, whey), animal (e.g., meat, fish), cereal (e.g., rice, corn), vegetable (e.g., soy), or combinations thereof. The proteins for use herein can also include, or be entirely or partially replaced by, free amino acids known for use in nutritional compositions, non-limiting examples of which include tryptophan, glutamine, tyrosine, methionine, cysteine, arginine, and combinations thereof. Other (non-protein) amino acids typically added to nutritional compositions include carnitine and taurine. In some cases, the D-forms of the amino acids are considered as nutritionally equivalent to the L-forms, and isomer mixtures are used to lower cost (for example, D,L-methionine).

Fats suitable for use in the compositions of the present disclosure include coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils, long-chain polyunsaturated fatty acids such as arachidonic acid (ARA), docosahexaenoic acid (DHA), and eicosapentaenoic acid (EPA), and combinations thereof.

In addition to these food grade oils, structured lipids may be incorporated into the nutritional compositions if desired. Structured lipids are known in the art, descriptions of which can be found in INFORM, Vol. 8, no. 10, page 1004, Structured lipids allow fat tailoring (October 1997); and U.S. Pat. No. 4,871,768, the latter description of which is incorporated herein by reference. Structured lipids are predominantly triacylglycerols containing mixtures of medium and long chain fatty acids on the same glycerol nucleus. Structured lipids are also described in U.S. Pat. No. 6,160,007, which is also incorporated herein by reference.

In some embodiments, carbohydrates in addition to maltodextrin may be included in the nutritional compositions such that the compositions include 2, 3, 4, or even more carbohydrate components. Additional carbohydrates suitable for use in the compositions of the present disclosure may be simple, complex, or variations or combinations thereof. Non-limiting examples of suitable carbohydrates include hydrolyzed or modified starch or cornstarch, corn syrup, corn syrup solids, rice-derived carbohydrate, glucose, fructose, lactose, high fructose corn syrup, indigestible oligosaccharides (e.g., fructooligosaccharides), soluble or insoluble fiber, honey, sugar alcohols (e.g., maltitol, erythritol, sorbitol), and combinations thereof.

The concentration or amount of fat, protein, and carbohydrate (including maltodextrin plus any additional carbohydrate) in the compositions of the present disclosure may vary considerably depending upon the particular product form (e.g., solid, liquid, powder) and the various other formulations and targeted dietary needs. These macronutrients are most typically formulated within any of the caloric ranges (embodiments A-F) described in the following table (each numerical value is preceded by the term "about").

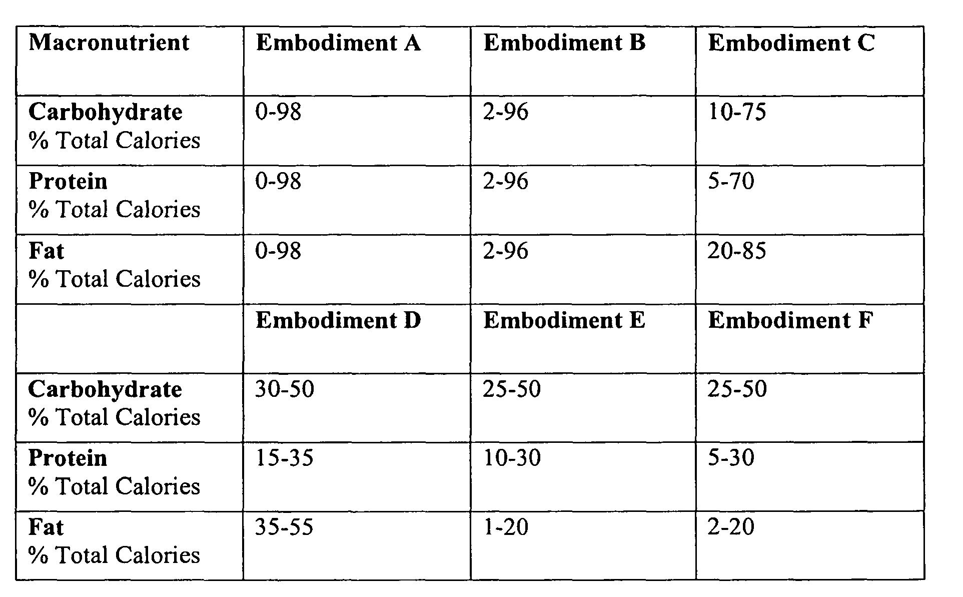

When the nutritional composition is an infant formula, the nutritional composition includes those embodiments in which the protein component may comprise from about 7.5% to about 25% of the caloric content of the nutritional composition; the carbohydrate component may comprise from about 35% to about 50% of the total caloric content of the nutritional composition; and the lipid component may comprise from about 30% to about 60% of the total caloric content of the nutritional composition. These ranges are provided as examples only, and are not intended to be limiting. Additional suitable ranges are noted in the following table (each numerical value is preceded by the term "about").

| **Nutrient** | **Embodiment G** | **Embodiment H** | **Embodiment I** |
|---|---|---|---|
| **Carbohydrates:** | 20-85 | 30-60 | 35-55 |
| % total calories | | | |
| **Lipid:** | 5-70 | 20-60 | 25-50 |
| % total calories | | | |
| **Protein:** | 2-75 | 5-50 | 7-40 |
| % total calories | | | |

### Optional Ingredients

The nutritional compositions may further comprise other optional components that may modify the physical, chemical, aesthetic or processing characteristics of the products or serve as pharmaceutical or additional nutritional components when used in a targeted population. Many such optional ingredients are known or otherwise suitable for use in nutritional products and may also be used in the compositions herein, provided that such optional ingredients are safe and effective for administration and are compatible with the essential and other selected components in the compositions.

Non-limiting examples of such other optional ingredients include preservatives, antioxidants, buffers, pharmaceutical actives, sweeteners, colorants, flavors, flavor enhancers, thickening agents and stabilizers, emulsifying agents, lubricants, and so forth.

The nutritional compositions may further include one or more minerals, non-limiting examples of which include phosphorus, sodium, chloride, magnesium, manganese, iron, copper, zinc, iodine, calcium, potassium, chromium, chromium picolinate, molybdenum, selenium, and combinations thereof.

The nutritional compositions may also include one or more vitamins, non-limiting examples of which include carotenoids (e.g., beta-carotene, zeaxanthin, lutein, lycopene), biotin, choline, inositol, folic acid, pantothenic acid, choline, vitamin A, thiamine (vitamin B1), riboflavin (vitamin B2), niacin (vitamin B3), pyridoxine (vitamin B6), cyanocobalamin (vitamin B12), ascorbic acid (vitamin C), vitamin D, vitamin E, vitamin K, and various salts, esters or other derivatives thereof, and combinations thereof.

### Manufacture

The nutritional compositions of the present disclosure may be prepared by any known or otherwise effective manufacturing technique for preparing the selected product solid or liquid form. Many such techniques are known for any given product form such as nutritional liquids or powders or nutritional bars and can easily be applied by one of ordinary skill in the art to the nutritional compositions described herein.

The nutritional compositions of the present disclosure can therefore be prepared by any of a variety of known or otherwise effective formulation or manufacturing methods. These methods most typically involve the initial formation of an aqueous slurry containing carbohydrates, proteins, lipids, stabilizers or other formulation aids, vitamins, minerals, or combinations thereof. The slurry is emulsified, pasteurized, homogenized, and cooled. Various other solutions, mixtures, or other materials may be added to the resulting emulsion before, during, or after further processing. This emulsion can then be further diluted, heat-treated, and packaged to form a ready-to-feed or concentrated liquid, or it can be heat-treated and subsequently processed and packaged as a reconstitutable powder, e.g., spray dried, dry mixed, agglomerated.

Other suitable methods for making nutritional compositions are described, for example, in U.S. Pat. No. 6,365,218 (Borschel, et al.), U.S Patent 6,589,576 (Borschel, et al.), U.S. Pat. No. 6,306,908 (Carlson, et al.), U.S. Patent Application 20030118703 A1 (Nguyen, et al.), which descriptions are incorporated herein by reference.

The solid matrix nutritional compositions may be manufactured using cold extrusion technology as is known in the art. To prepare such compositions, typically all of the powdered components will be dry blended together. Such constituents typically include the proteins, vitamin premixes, certain carbohydrates, and so forth. The fat soluble components are then blended together and mixed with the powdered premix above. Finally any liquid components are then mixed into the composition, forming a plastic like composition or dough.

The process above is intended to give a plastic mass, which can then be shaped, without further physical or chemical changes occurring, by the procedure known as cold forming or extrusion. In this process, the plastic mass is forced at relatively low pressure through a die, which confers the desired shape, and the resultant exudate is then cut off at an appropriate position to give products of the desired weight.

The mass may, for example, be forced through a die of small cross-section to form a ribbon, which is carried on a belt moving at a predetermined speed under a guillotine type cutter which operates at regular intervals. The cutter, in this case, generally consists of a sharpened blade so adjusted that it cuts through the ribbon but not the underlying belt, but may also consist of a wire. In both cases, the principle is the same; the cutting process occurs at intervals that permit the moving ribbon to be cut into pieces of equivalent weight and dimensions. Generally, this is achieved by timing the cutting strokes and maintaining belt speed at an appropriate level, but there also exist computer controlled versions of this mechanism which offer greater versatility. Alternatively, the mass may be forced through a die of large cross-section and then cut at die level into slices by an oscillating knife or wire, which drop onto a moving belt and are thus transported away. The mass may also be extruded as a sheet, which is then cut with a stamp type cutter into shapes that are appropriate, such as a cookie type cutter. Finally, the mass may also be forced into chambers on a rotary die equipped with an eccentric cam that forces the thus-formed material out of the chamber at a certain point in a rotation of the cylindrical die.

After shaping, the formed product is moved by a transfer belt or other type of material conveyor to an area where it may be further processed or simply packaged. In general, a nutritional bar of the type described can be enrobed (coated) in a material that may be chocolate, a compound chocolate coating, or some other type of coating material. The coating material typically consists of a fat that is solid at room temperature, but that is liquid at temperatures in excess of e.g. 31°C, together with other materials that confer the organoleptic attributes. The coating is thus applied to the bar while molten, by permitting the bar to pass through a falling curtain of liquid coating, at the same time passing over a plate or rollers which permit coating to be applied to the under surface of the bar, and excess coating is blown off by means of air jets. Finally, the enrobed bar passes through a cooling tunnel where refrigerated air currents remove heat and cause the coating to solidify.

The solid matrix nutritional embodiments for use herein may also be manufactured through a baked application or heated extrusion to produce solid product forms such as cereals, cookies, crackers, and similar other product forms. One knowledgeable in the nutrition manufacturing arts is able to select one of the many known or otherwise available manufacturing processes to produce the desired final product.

Numerous types of packaging are readily available and known to one practicing the art. Desirable packaging characteristics include: effective protection against impact, light, and heat; ease of opening; and efficient sealing for storage stability.

### Methods of Use

The nutritional compositions of the present disclosure may be orally administered to a subject (e.g., infant or child) to enhance cognition and/or improve memory in the subject. Specifically, it has been discovered that an enhancement in cognition and/or an improvement in memory in an infant or child can be achieved by administering maltodextrin, for example, in the form of a nutritional composition of the present disclosure, prior to a cognitive task (e.g., a problem-solving task or memory task). For example, the nutritional composition including maltodextrin can be orally administered to a child such that the intake of maltodextrin is from about 0.5 g/kg body weight/dose to about 2.5 g/kg body weight/dose and then the child can be challenged with a memory task such as recalling a list of items previously given to him/her. By administering the nutritional composition including maltodextrin, the memory of the child is improved.

Typically, the cognitive enhancement and/or memory improvement is shown more than one hour after administration of the nutritional composition. In one embodiment, the cognitive enhancement and/or memory improvement is shown at least two hours after administration of the nutritional composition, including at least three hours after administration, and including at least four hours after administration.

Thus, in one embodiment, the present disclosure is directed to a method of improving memory in an infant and/or child. The method comprises administering to the infant or child the nutritional composition such that the intake of maltodextrin is from about 0.5 g/kg body weight/dose to about 2.5 g/kg body weight/dose prior to challenging the subject with a memory task. An improvement in memory can be, for example, an improvement in hippocampal-dependent tasks such as spatial memory and related forms of learning and memory. An improvement in memory can be measured using any suitable technique known in the art such as, for example, the British ability scale, Cognitive Performace Test (CPT) or Story recall.

In another embodiment, the present disclosure is directed to a method of enhancing cognition in an infant and/or child. The method comprises administering to the infant or child the nutritional composition such that the intake of maltodextrin is from about 0.5 g/kg body weight/dose to about 2.5 g/kg body weight/dose prior to challenging the subject with a cognitive task. The terms "cognition," "cognitive," "cognitive function," and "cognitive task" as used herein are interchangeable and refer to a range of functions: intelligence (fluid, crystallized), creativity (generate and open to new ideas, perceive new relationships), memory (encoding, storage, retrieval), executive functions (dealing with novelty, planning & implementation, monitoring performance, vigilance, inhibition of task irrelevant info), cognitive resources (speed of information processing, working memory capacity, attentional capacity), and the like. An enhancement in cognition can include an improvement or enhancement of cognitive development. The term "cognitive development" as used herein refers to the development of cognition (cognitive function) over time. The quality of cognition (cognitive function) in a human can be assessed by tests measuring cognitive performance. As cognition (cognitive function) of a growing infant or child develops over time with brain development, measuring the cognitive performance over time for a given infant or child, provides a measure of cognitive development. For example, for children aged 6-9 years, cognitive development is generally recognized by: a spurt in information processing capacity and/or executive functions, and development of the frontal lobes of the brain (involved in executive functions). These growth spurts may also occur at other periods during childhood (e.g. 0-2 years and mid-teens).

Typically, the effects resulting from administration of the nutritional composition, e.g., enhancement in cognition and/or improvement in memory in the infant or child, is an acute rather than chronic effect. As used herein, the term "acute effect" means the cognitive enhancement and/or memory improvement is temporary. In other words, the enhancement in cognition and/or the improvement in memory achieved following administration of the nutritional compositions of the present disclosure will diminish to approximate levels present prior to administration of the nutritional composition after a certain period of time, rather than remaining as a permanent increase in the level of cognition and/or memory. The time at which the enhancement in cognition and/or improvement in memory has diminished may vary, but may be less than 1 day after administration of the composition or less than 10 hours after administration of the composition. Thus, the nutritional composition may be administered once per day to produce a one time enhancement in cognition and/or improvement in memory, or alternately may be administered two or more times per day to produce multiple instances of enhancement in cognition and/or improvement in memory in the same day.

Thus, in one embodiment, the present disclosure is directed to a method of enhancing cognition and/or improving memory in an infant or child. The method comprises administering to the infant or child once per day the nutritional composition such that the intake of maltodextrin is from about 0.5 g/kg body weight/dose to about 2.5 g/kg body weight/dose. It will be recognized that the infant or child may be administered a second nutritional composition, which may or may not comprise maltodextrin, on the same day as the nutritional composition of the present disclosure.

In another embodiment, the present disclosure is directed to a method of enhancing cognition and/or improving memory in an infant and/or child receiving multiple feedings per day. The method comprises administering to the infant and/or child during a first feeding the nutritional composition such that the intake of maltodextrin is from about 0.5 g/kg body weight/dose to about 2.5 g/kg body weight/dose. Administration of the nutritional composition during the first feeding produces an effect in the infant or child more than one hour after administration of the nutritional composition, the effect being selected from the group consisting of enhanced cognition, improved memory, and combinations thereof. The infant or child is then administered the nutritional composition during a second feeding such that the intake of maltodextrin is from about 0.5 g/kg body weight/dose to about 2.5 g/kg body weight/dose. The second feeding is administered after the effect produced by the first feeding has diminished. The enhancement in cognition and/or improvement in memory has diminished when the level of cognition and/or memory, assessed as described herein, begins to decrease to levels present prior to administration of the nutritional composition. The time at which the enhancement in cognition and/or improvement in memory has diminished may vary, but typically is less than 1 day after administration of the composition, and more typically less than 10 hours after administration of the composition. Thus, the second feeding is administered after the effect produced by the first feeding has diminished, which may be, for example, at least four hours, more typically at least five hours, or at least six hours after the first feeding.

### EXAMPLES

The following Examples illustrate specific embodiments and/or features of the compositions and methods of the present disclosure. The Examples are given solely for the purpose of illustration and are not to be construed as limitations, as many variations thereof are possible without departing from the spirit and scope of the disclosure.

### Example 1

In this Example, the effect of maltodextrin and sucrose on memory of Spraque-Dawley (SD) rats was tested.

Thirty-six male SD rats were assigned to four treatment groups: Group A (n=9); Group B (n=9); Group C (n=9); and Group D (n=9). After weaning (postnatal day 21), the rats were group-housed and maintained on a 12 hour:12 hour light:dark cycle. The behavioral procedures described below were performed during the light phase of the cycle from postnatal day 27 to postnatal day 34. Food and water was available ad *libitum* in the home cage. Twelve hours of fasting was required before testing. A specific liquid solution (test solution) was administered intragastrically (i.g.) to the rats in each group at a dose of 1g/kg body weight before the acquisition and testing sessions. The control group received a volume of water per body weight equivalent to the volume received by the experimental groups. The groups were: A) Sucrose; B) Maltodextrin DE 19-24; C) Maltodextrin DE 8-10; and D) Water.

### Behavioral Procedure

Testing took place in an opaque plastic chamber (35 cm x 41 cm x 50 cm). Two pairs of stimuli objects were used (Pair 1 and Pair 2). The objects of pairs 1 and 2 were of different shape, color and/or material. Velcro was used to attach each object to the floor of the testing boxes (9 cm apart), both to ensure correct positioning of the object and to prevent the rats from displacing the object during testing. A video camera mounted above the chamber allowed recording of the sessions. Overhead lighting illuminated the testing area reducing room context information.

A 3-day novel-object-recognition procedure was conducted (see Figure 1). During the first day, the habituation session took place. Specifically, all of the groups were acclimated to the testing room (45 minutes) and they were habituated to the empty chamber, allowing the rats 5 minutes for exploration.

The acquisition session was done 24 hour later. All of the groups received the intragastric test solution and were exposed to two object pairs (Pair 1 and Pair 2) at time intervals of 30 and 180 minutes after administration. Each rat explored each pair of identical objects for a period of 15 minutes.

The testing session took place 24 hours after the acquisition session. The test solution was administered again to all groups and two recognition memory tests were applied at the same time intervals used in the acquisition session (the first pair of objects at 30 minutes and the second pair of objects at 180 minutes after administration of the test solution). In this session, one of the two objects in the pair was novel. The rat was allowed to explore the objects until it accumulated 30 seconds of contact time with the objects (i.e., nose within 2 cm of objects and vibrissae moving). The time that the rat spent exploring the novel and the familiar object was recorded. Both the novel object and the position in the chamber were counterbalanced within each group.

### Statistical analysis

Raw data were analyzed using mixed ANOVA, with novelty being the within-subject factor and group being the between-subject factor. The difference between the time spent looking at the novel and the familiar object was calculated for each animal as a discrimination index of object recognition. The data were analyzed by one-way ANOVA followed by LSD post-hoc comparison.

### Results

### Acquisition Session

In the acquisition session two pairs of identical objects were presented to the animals. The test is valid if the animals do not show any preference between the two identical objects. This way, it can be assumed that when the new object is presented, the only reason to explore this object more is novelty and not the position of the object or any other cue. There was no preference for one of the objects over the other in any of the groups when exploring the two pairs of objects (data not shown).

### Testing Session

All the groups explored longer the novel object both at 30 minutes and 180 minutes after the administration of the test solution, thus confirming recognition memory. Figure 2 shows the mean (±SEM) discrimination index (difference score between the time spent exploring the new object and the familiar objects) at both time points. No differences among groups were found at 30 minutes (Figure 2A) after the administration, but significant differences were found at 180 minutes (Figure 2B). Specifically, Groups B (Maltodextrin DE 19-24) and C (Maltodextrin DE 8-10) spent significantly longer time exploring the novel object as compared to Group A (Sucrose), while Group C (Maltodextrin DE 8-10) was marginally different from Group D (Water) (p=0.07). There were no differences between the rest of the groups.

### Conclusion

These results demonstrate that maltodextrin having a DE of 8-10 or a DE of 19-24 has a beneficial effect on memory of the rats 180 minutes after administration. Administration of sucrose did not result in any effects under the conditions of this experiment.

### Example 2

In this Example, the effect on memory of maltodextrin, sucrose and two slow-released carbohydrates: γ-cyclodextrin and sucromalt was tested in SD rats.

Forty male SD rats were assigned to four treatment groups: Group E, γ-Cyclodextrin (n=10); Group F Sucromalt (n=10); Group G Maltodextrin DE 8-10 (n=10); and Group H Sucrose (n=10). The experimental protocol was as described in Example 1 except that the behavioral protocol was done from postnatal day 24 to postnatal day 32. The methods were also performed as described in Example 1.

### Results

### Acquisition session

When the rats were presented the two pairs of identical objects during the acquisition session, there was no preference of one object over the other (data not shown).

### Testing session

Figure 3 shows the mean (±SEM) difference score between the time exploring the novel object and the familiar object 30 minutes (Figure 3A) and 180 minutes (Figure 3B) after the administration of the test solution. There were no differences among groups at 30 minutes. However, at 180 minutes, group G (Maltodextrin DE 8-10) spent more time exploring the novel object (p<.01) than the rest of the groups.

### Conclusion

These results demonstrate that maltodextrin having a DE of 8-10 has a beneficial effect on memory of the rats 180 minutes after administration. The slow release carbohydrates γ-cyclodextrin and sucromalt showed no effects on memory under the conditions of this experiment.

### Example 3

The purpose of this Example was twofold: 1) to compare maltodextrin with glucose or water and 2) to study if maltodextrin should be given twice, before acquisition and before testing, to exert the effect on memory that was found in Examples 1 and 2.

Forty-eight male SD rats were assigned to five treatment groups: Group I, Water (n=8); Group J, Glucose (n=10); Group K, Maltodextrin DE 8-10 given as in Example 1 and 2, before acquisition and testing (n=10); Group L, Maltodextrin DE 8-10 given only before acquisition (n=10); Group M, Maltodextrin DE 8-10 given only before testing (n=10). The experimental protocol was as described in Example 1 except that the behavioral protocol was done from postnatal day 26 to postnatal day 34. The methods were also performed as described in Example 1.

### Results

### Acquisition session

As in Example 1 and 2, the rats did not show any preference for the two identical pair of objects in the acquisition section (data not shown)

### Testing session

All the groups recognized the new object and spent more time looking at it, both 30 or 180 minutes after the administration with the test solutions

Figure 4 shows the mean (±SEM) difference score between the time exploring the novel object and the familiar object 30 minutes (Figure 4A) and 180 minutes (Figure 4B) after the administration of the test solution. As in previous the examples, there were no differences among groups at 30 minutes. However, at 180 minutes, only group K (Maltodextrin DE 8-10 given before acquisition and testing) spent more time exploring the novel object (p<.01) than the rest of the groups. These results indicate that maltodextrin should be given both before the acquisition and before the testing sessions to achieve an acute effect on memory.

### Conclusions

In summary, the results showed no effect of any of the carbohydrates tested 30 minutes after the administration of the test solutions, whereas at 180 minutes maltodextrin showed a differential effect. In spite of the fact that all of the groups recall the familiar object, the results indicated a beneficial effect on memory of the groups that received maltodextrin independently of the DE. Other simple carbohydrates, namely the monosaccharide glucose and the disaccharide sucrose, did not showed any effect under the experimental conditions of this assay. In addition, there were no effects on memory of slow release carbohydrates, specifically γ-cyclodextrin or sucromalt. The test was carried out in two sessions: acquisition, when the identical objects were presented, and testing, when the novel and familiar objects were presented. To find an acute effect on memory, maltodextrin should be given both before the acquisition and before the testing sessions.

### Examples 4-9

Examples 4-9 illustrate nutritional compositions of the present disclosure including maltodextrin, the ingredients of which are listed in the table below.

All ingredient amounts are listed as kg per 1000 kg batch of product, unless otherwise specified. The compositions are prepared by forming at least three separate slurries (e.g., carbohydrate-mineral slurry, protein-in-water slurry, protein-in-fat slurry) which are then blended together, heat-treated, and standardized. The resulting composition is then flavored, aseptically packaged into plastic bottles or retort sterilized.

| **Ingredient** | **Example 4** | **Example 5** | **Example 6** | **Example 7** | **Example 8** | **Example 9** |
|---|---|---|---|---|---|---|
| Water | Q.S. | Q.S. | Q.S. | Q.S. | Q.S. | Q.S. |
| Maltodextrin (Glucidex 29) | 352.0 | 465.3 | 510.2 | 375.4 | 550.2 | 426.3 |
| Sucrose | 212.5 | 99.2 | 54.3 | 189.1 | 14.3 | 138.2 |
| Milk Protein Concentrate (80%) | 120.0 | 116.2 | 113.9 | 114.4 | 118.1 | 121.2 |
| Whey Protein Concentrate | 25.5 | 28.2 | 27.7 | 24.3 | 25.1 | 29.0 |
| Isolated Soy Protein (Supro 1610) | 20.5 | 21.6 | 24.4 | 27.3 | 22.8 | 15.8 |
| Soy Oil | 75.8 | 76.7 | 79.6 | 77.8 | 74.7 | 80.1 |
| High Oleic Sunflower Oil | 69.2 | 68.3 | 65.4 | 67.2 | 70.3 | 64.9 |
| Medium Chain Triglycerides (MCT Oil) | 25.2 | 24.1 | 23.5 | 20.7 | 30.2 | 28.5 |
| Fructo-oligosaccharide (FOS) powder | 22.0 | 21.5 | 20.9 | 23.4 | 22.1 | 20.0 |
| Potassium citrate | 6.5 | 6.2 | 7.0 | 5.9 | 6.1 | 7.2 |
| Flavors | 50.1 | 47.6 | 40.2 | 55.3 | 41.6 | 46.5 |
| Magnesium phosphate dibasic | 5.0 | 4.8 | 5.6 | 5.2 | 4.9 | 4.2 |
| Potassium chloride | 4.0 | 4.2 | 3.8 | 3.6 | 4.1 | 4.9 |
| Sodium chloride | 3.7 | 3.5 | 3.9 | 4.1 | 3.6 | 2.9 |
| Tricalcium phosphate | 3.2 | 3.0 | 2.8 | 2.9 | 3.2 | 3.0 |
| Vitamin Mineral Taurine Premix | 2.4 | 2.4 | 2.4 | 2.4 | 2.4 | 2.4 |
| Docosahexanoic | 2.0 | 2.0 | 2.0 | 2.0 | 2.0 | 2.0 |
| Acid Powder | | | | | | |
| Choline chloride | 1.5 | 1.5 | 1.6 | 1.5 | 1.1 | 1.3 |
| Potassium phosphate, monobasic | 1.5 | 1.5 | 1.5 | 1.5 | 1.5 | 1.5 |
| Calcium carbonate | 1.3 | 1.3 | 1.3 | 1.3 | 1.3 | 1.3 |
| Potassium phosphate, dibasic | 1.1 | 1.1 | 1.1 | 1.1 | 1.1 | 1.1 |
| Ascorbic acid | 0.834 | 0.834 | 0.834 | 0.834 | 0.834 | 0.834 |
| Arachidonic Acid Powder | 0.645 | 0.645 | 0.645 | 0.645 | 0.645 | 0.645 |
| Ascorbyl palmitate | 0.481 | 0.481 | 0.481 | 0.481 | 0.481 | 0.481 |
| Vitamin ADEK Premix | 0.169 | 0.169 | 0.169 | 0.169 | 0.169 | 0.169 |
| Lactobacillus acidophilus | 0.1 | 0.1 | 0.1 | 0.1 | 0.1 | 0.1 |
| Tocopherol | 0.08 | 0.08 | 0.08 | 0.08 | 0.08 | 0.08 |
| dl-alpha tocopherylacetate | 0.047 | 0.047 | 0.047 | 0.047 | 0.047 | 0.047 |
| Bifidobacterium lactis | 0.035 | 0.035 | 0.035 | 0.035 | 0.035 | 0.035 |
| Vitamin A palmitate | 0.001 | 0.001 | 0.001 | 0.001 | 0.001 | 0.001 |
| Potassium iodide | 0.000085 | 0.000085 | 0.000085 | 0.000085 | 0.000085 | 0.000085 |

### Examples 10-15

Examples 10-15 illustrate nutritional compositions of the present disclosure including maltodextrin as the sole source of carbohydrate, the ingredients of which are listed in the table below.

All ingredient amounts are listed as kg per 1000 kg batch of product, unless otherwise specified. The compositions are prepared by forming at least three separate slurries (e.g., carbohydrate-mineral slurry, protein-in-water slurry, protein-in-fat slurry) which are then blended together, heat-treated, and standardized. The resulting composition is then flavored, aseptically packaged into plastic bottles or retort sterilized.

| **Ingredient** | **Example 10** | **Example 11** | **Example 12** | **Example 13** | **Example 14** | **Example 15** |
|---|---|---|---|---|---|---|
| Water | Q.S. | Q.S. | Q.S. | Q.S. | Q.S. | Q.S. |
| Maltodextrin (Glucidex 29) | 565.0 | 650.0 | 520.6 | 775.4 | 584.2 | 426.3 |
| Milk Protein Concentrate (80%) | 120.0 | 116.2 | 113.9 | 114.4 | 118.1 | 121.2 |
| Whey Protein Concentrate | 25.5 | 28.2 | 27.7 | 24.3 | 25.1 | 29.0 |
| Isolated Soy Protein (Supro 1610) | 20.5 | 21.6 | 24.4 | 27.3 | 22.8 | 15.8 |
| Soy Oil | 75.8 | 76.7 | 79.6 | 77.8 | 74.7 | 80.1 |
| High Oleic Sunflower Oil | 69.2 | 68.3 | 65.4 | 67.2 | 70.3 | 64.9 |
| Medium Chain Triglycerides (MCT Oil) | 25.2 | 24.1 | 23.5 | 20.7 | 30.2 | 28.5 |
| Fructo-oligosaccharide (FOS) powder | 22.0 | 21.5 | 20.9 | 23.4 | 22.1 | 20.0 |
| Potassium citrate | 6.5 | 6.2 | 7.0 | 5.9 | 6.1 | 7.2 |
| Flavors | 50.1 | 47.6 | 40.2 | 55.3 | 41.6 | 46.5 |
| Magnesium phosphate dibasic | 5.0 | 4.8 | 5.6 | 5.2 | 4.9 | 4.2 |
| Potassium chloride | 4.0 | 4.2 | 3.8 | 3.6 | 4.1 | 4.9 |
| Sodium chloride | 3.7 | 3.5 | 3.9 | 4.1 | 3.6 | 2.9 |
| Tricalcium phosphate | 3.2 | 3.0 | 2.8 | 2.9 | 3.2 | 3.0 |
| Vitamin Mineral Taurine Premix | 2.4 | 2.4 | 2.4 | 2.4 | 2.4 | 2.4 |
| Docosahexanoic Acid Powder | 2.0 | 2.0 | 2.0 | 2.0 | 2.0 | 2.0 |
| Choline chloride | 1.5 | 1.5 | 1.6 | 1.5 | 1.1 | 1.3 |
| Potassium phosphate, monobasic | 1.5 | 1.5 | 1.5 | 1.5 | 1.5 | 1.5 |
| Calcium carbonate | 1.3 | 1.3 | 1.3 | 1.3 | 1.3 | 1.3 |
| Potassium phosphate, dibasic | 1.1 | 1.1 | 1.1 | 1.1 | 1.1 | 1.1 |
| Ascorbic acid | 0.834 | 0.834 | 0.834 | 0.834 | 0.834 | 0.834 |
| Arachidonic Acid Powder | 0.645 | 0.645 | 0.645 | 0.645 | 0.645 | 0.645 |
| Ascorbyl palmitate | 0.481 | 0.481 | 0.481 | 0.481 | 0.481 | 0.481 |
| Vitamin ADEK Premix | 0.169 | 0.169 | 0.169 | 0.169 | 0.169 | 0.169 |
| Lactobacillus acidophilus | 0.1 | 0.1 | 0.1 | 0.1 | 0.1 | 0.1 |
| Tocopherol | 0.08 | 0.08 | 0.08 | 0.08 | 0.08 | 0.08 |
| dl-alpha tocopheryl acetate | 0.047 | 0.047 | 0.047 | 0.047 | 0.047 | 0.047 |
| Bifidobacterium lactis | 0.035 | 0.035 | 0.035 | 0.035 | 0.035 | 0.035 |
| Vitamin A palmitate | 0.001 | 0.001 | 0.001 | 0.001 | 0.001 | 0.001 |
| Potassium iodide | 0.000085 | 0.000085 | 0.000085 | 0.000085 | 0.000085 | 0.000085 |

## Claims

1. A method of enhancing cognition in an infant or child, the method comprising administering to the infant or child a nutritional composition comprising maltodextrin, wherein the nutritional composition is administered such that the intake of maltodextrin is from about 0.5 g/kg body weight/dose to about 2.5 g/kg body weight/dose.

2. The method as set forth in claim 1 wherein the composition comprises from about 30% to about 85% (by weight nutritional composition) maltodextrin.

3. The method as set forth in claim 1 wherein the composition is administered such that the intake of maltodextrin is from about 0.6 g/kg body weight/dose to about 2.0 g/kg body weight/dose.

4. The method as set forth in claim 1 wherein the infant or child shows enhanced cognition more than one hour after administration of the nutritional composition.

5. The method as set forth in claim 1 wherein the infant or child shows enhanced cognition at least 3 hours after administration of the nutritional composition.

6. A method of improving memory in an infant or child, the method comprising administering to the infant or child a nutritional composition comprising maltodextrin, wherein the nutritional composition is administered such that the intake of maltodextrin is from about 0.5 g/kg body weight/dose to about 2.5 g/kg body weight/dose.

7. The method as set forth in claim 6 wherein the composition comprises from about 30% to about 85% (by weight nutritional composition) maltodextrin.

8. The method as set forth in claim 6 wherein the composition is administered such that the intake of maltodextrin is from about 0.6 g/kg body weight/dose to about 2.0 g/kg body weight/dose.

9. The method as set forth in claim 6 wherein the infant or child shows improved memory more than one hour after administration of the nutritional composition.

10. The method as set forth in claim 6 wherein the infant or child shows improved memory at least 3 hours after administration of the nutritional composition.

11. A method of enhancing cognition and/or improving memory in an infant or child, the method comprising administering to the infant or child a nutritional composition comprising maltodextrin, wherein maltodextrin is the sole source of carbohydrate in the nutritional composition, and wherein the nutritional composition is administered such that the intake of maltodextrin is from about 0.5 g/kg body weight/dose to about 2.5 g/kg body weight/dose.

12. The method as set forth in claim 11 wherein the composition comprises from about 30% to about 85% (by weight nutritional composition) maltodextrin.

13. The method as set forth in claim 11 wherein the composition is administered such that the intake of maltodextrin is from about 0.6 g/kg body weight/dose to about 2.0 g/kg body weight/dose.

14. A method of enhancing cognition and/or improving memory in an infant or child, the method comprising administering to the infant or child once per day a nutritional composition comprising maltodextrin, wherein the nutritional composition is administered such that the intake of maltodextrin is from about 0.5 g/kg body weight/dose to about 2.5 g/kg body weight/dose.

15. The method as set forth in claim 14 wherein the composition comprises from about 30% to about 85% (by weight nutritional composition) maltodextrin.

16. The method as set forth in claim 14 wherein the composition is administered such that the intake of maltodextrin is from about 0.6 g/kg body weight/dose to about 2.0 g/kg body weight/dose.

17. The method as set forth in claim 14 wherein nutritional composition comprising maltodextrin is a first nutritional composition, and the infant and/or child is further administered a second nutritional composition on the same day as the first nutritional composition.

18. A method of enhancing cognition and/or improving memory in an infant or child receiving multiple feedings per day, the method comprising:
administering to the infant or child during a first feeding a nutritional composition comprising maltodextrin, wherein the nutritional composition is administered such that the intake of maltodextrin is from about 0.5 g/kg body weight/dose to about 2.5 g/kg body weight/dose, and administration of the nutritional composition during the first feeding produces an effect in the infant or child more than one hour after administration of the nutritional composition, the effect being selected from the group consisting of enhanced cognition, improved memory, and combinations thereof; and
administering to the infant or child the nutritional composition during a second feeding such that the intake of maltodextrin is from about 0.5 g/kg body weight/dose to about 2.5 g/kg body weight/dose, wherein the second feeding is administered after the effect produced by the first feeding has diminished.

19. The method of claim 18, wherein the second feeding is administered at least four hours after the first feeding.

20. The method of claim 19, wherein the second feeding is administered at least five hours after the first feeding.

## Amended claims

### Amended claims in accordance with Rule 137(2) EPC.

**1.** Use of maltodextrin for manufacture of a nutritional composition for enhancing cognition and/or improving memory in an infant or child by administration of the nutritional composition to the infant or child, **characterized in that** the administration is such that the intake of maltodextrin is from about 0.5 g/kg body weight/dose to about 2.5 g/kg body weight/dose.

**2.** The use as set forth in claim 1 **characterized in that** the composition comprises from about 30% to about 85% (by weight nutritional composition) maltodextrin.

**3.** The use as set forth in claim 1 **characterized in that** the administration is such that the intake of maltodextrin is from about 0.6 g/kg body weight/dose to about 2.0 g/kg body weight/dose.

**4.** The use as set forth in claim 1 **characterized in that** the infant or child shows enhanced cognition and/or improved memory more than one hour after administration of the nutritional composition.

**5.** The use as set forth in claim 1 **characterized in that** the infant or child shows enhanced cognition and/or improved memory at least 3 hours after administration of the nutritional composition.

**6.** The use as set forth in any one of claims 1 to 3, **characterized in that** maltodextrin is the sole source of carbohydrate in the nutritional composition.

**7.** The use as set forth in any one of claims 1 to 3 **characterized in that** the administration occurs once per day.

**8.** The use as set forth in claim 7 **characterized in that** nutritional composition comprising maltodextrin is a first nutritional composition, and the infant and/or child is further administered a second nutritional composition on the same day as the first nutritional composition.

**9.** The use as set forth in any one of claims 1 to 3, **characterized in that** infant or child receives multiple feedings per day, and **characterized in that**
administration to the infant or child during a first feeding of the nutritional composition comprising maltodextrin is such that the intake of maltodextrin is from about 0.5 g/kg body weight/dose to about 2.5 g/kg body weight/dose, and administration of the nutritional composition during the first feeding produces an effect in the infant or child more than one hour after administration of the nutritional composition, the effect being selected from the group consisting of enhanced cognition, improved memory, and combinations thereof; and
administration to the infant or child the nutritional composition during a second feeding is such that the intake of maltodextrin is from about 0.5 g/kg body weight/dose to about 2.5 g/kg body weight/dose, wherein the second feeding is administered after the effect produced by the first feeding has diminished.

**10.** The use of claim 9, **characterized in that** the second feeding is administered at least four hours after the first feeding.

**11.** The use of claim 10, **characterized in that** the second feeding is administered at least five hours after the first feeding.
